# EUROPEAN PATENT SPECIFICATION

(11) **EP 1 930 410 B1**
(45) Date of publication and mention of the grant of the patent: **22.06.2011**
(21) Application number: 06797012.9
(22) Date of filing: 23.08.2006
(51) Int. Cl.: C12N 1/21, C12N 15/09, C12P 13/14, C12Q 1/68

(54) **L-GLUTAMIC ACID-PRODUCING BACTERIUM AND METHOD FOR PRODUCTION OF L-GLUTAMIC ACID**
L-GLUTAMINSÄURE PRODUZIERENDES BAKTERIUM UND VERFAHREN ZUR HERSTELLUNG VON L-GLUTAMINSÄURE
BACTERIE PRODUISANT DE L'ACIDE L-GLUTAMIQUE ET PROCEDE DE PRODUCTION DE L'ACIDE L-GLUTAMIQUE

(30) Priority: 26.08.2005 JP 2005245213
(43) Date of publication of application: 11.06.2008
(73) Proprietor: Ajinomoto Co., Inc., Tokyo 104-8315 (JP)
(72) Inventor: HIRANO, Seiko, Kawasaki-shi, Kanagawa 2108681 (JP); NAKAMURA, Jun, Kawasaki-shi, Kanagawa 2108681 (JP); ITO, Hisao, Kawasaki-shi, Kanagawa 2108681 (JP)
(74) Representative: HOFFMANN EITLE
(86) International application number: PCT/JP2006/317037
(87) International publication number: WO 2007/024011

(56) References cited:
- EP-A- 1 002 866
- EP-A- 1 174 508
- WO-A1-95/34672
- DE-A1- 10 128 510
- JP-A- 01 296 994
- KIMURA EIICHIRO ET AL: "Glutamate overproduction in Corynebacterium glutamicum triggered by a decrease in the level of a complex comprising DtsR and a biotin-containing subunit" BIOSCIENCE BIOTECHNOLOGY AND BIOCHEMISTRY, vol. 63, no. 7, July 1999 (1999-07), pages 1274-1278, XP002503233 ISSN: 0916-8451
- DATABASE PROTEIN [Online] 'Hypothetical protein predicted by Glimmer/Critica [Corynebacterium glutamicum ATCC 13032]', XP003003855 Database accession no. (CAF20999)
- KALINOWSKI J. ET AL.: 'The complete Corynebacterium glutamicum ATCC 13032 genome sequence and its impact on the production of L-aspartate-derived amino acids and vitamins' J. BIOTECHNOL. vol. 104, no. 1-3, 2003, pages 5 - 25, XP002371548
- NISHIYAMA T. ET AL.: 'The transition of research on industrial microorganism for L-glutamate overproduction using Coryneform bacteria' SCI. TECHNOL. JPN. vol. 19, no. 75, 2000, pages 18 - 21, XP003003856
- SCHAFER A. ET AL.: 'Small mobilizate multi-purpose cloning vectors derived from the Escherichia coli plasmids pK18 and pK19: selection of defined deletions in the chromosome of Corynebacterium glutamicum' GENE vol. 145, no. 1, 1994, pages 69 - 73, XP001093898

### SEQUENCE LISTING

<110> Ajinomoto Co., Inc.
<120> L-Glutamic acid-producing bacterium and method for production of L-glutamic acid
<130> C584-C7324
<150> JP2005-245213
   <151> 2005-08-26
<160> 17
<170> PatentIn version 3.1
<210> 1
   <211> 24
   <212> DNA
   <213> Artificial
<220>
   <223> sacB primer 1
<400> 1
   cgggatcctt tttaacccat caca 24
<210> 2
   <211> 29
   <212> DNA
   <213> Artificial
<220>
   <223> sacB primer 2
<400> 2
   gaagatcttc aaaaggttag gaatacggt 29
<210> 3
   <211> 23
   <212> DNA
   <213> Artificial
<220>
   <223> sacB primer 3
<400> 3
   ccttttgaag atcgaccagt tgg 23
<210> 4
   <211> 44
   <212> DNA
   <213> Artificial
<220>
   <223> sacB primer 4
<400> 4
   tacctggaat gctgttttcc cagggatcgc agtggtgagt aacc 44
<210> 5
   <211> 28
   <212> DNA
   <213> Artificial
<220>
   <223> sacB primer 5
<400> 5
   cctgggaaaa cagcattcca ggtattag 28
<210> 6
   <211> 23
   <212> DNA
   <213> Artificial
<220>
   <223> sacB primer 6
<400> 6
   tgcaggtcga ctctagagga tcc 23
<210> 7
   <211> 2014
   <212> DNA
   <213> Bacillus subtilis
<220>
   <221> CDS
   <222> (464).. (1885)
   <223> sacB
<400> 7
   gatccttttt aacccatcac atatacctgc cgttcactat tatttagtga aatgagatat      60
   tatgatattt tctgaattgt gattaaaaag gcaactttat gcccatgcaa cagaaactat     120
   aaaaaataca gagaatgaaa agaaacagat agatttttta gttctttagg cccgtagtct      180
   gcaaatcctt ttatgatttt ctatcaaaca aaagaggaaa atagaccagt tgcaatccaa     240
   acgagagtct aatagaatga ggtcgaaaag taaatcgcgc gggtttgtta ctgataaagc     300
   aggcaagacc taaaatgtgt aaagggcaaa gtgtatactt tggcgtcacc ccttacatat     360
   tttaggtctt tttttattgt gcgtaactaa cttgccatct tcaaacagga gggctggaag     420
   aagcagaccg ctaacacagt acataaaaaa ggagacatga acg atg aac atc aaa       475
                                                   Met Asn Ile Lys
                                                   1
   aag ttt gca aaa caa gca aca gta tta acc ttt act acc gca ctg ctg       523
   Lys Phe Ala Lys Gln Ala Thr Val Leu Thr Phe Thr Thr Ala Leu Leu
   5                   10                  15                  20
   gca gga ggc gca act caa gcg ttt gcg aaa gaa acg aac caa aag cca       571
   Ala Gly Gly Ala Thr Gln Ala Phe Ala Lys Glu Thr Asn Gln Lys Pro
                   25                  30                  35
   tat aag gaa aca tac ggc att tcc cat att aca cgc cat gat atg ctg       619
   Tyr Lys Glu Thr Tyr Gly Ile Ser His Ile Thr Arg His Asp Met Leu
               40                  45                  50
   caa atc cct gaa cag caa aaa aat gaa aaa tat caa gtt cct gaa ttc       667
   Gln Ile Pro Glu Gln Gln Lys Asn Glu Lys Tyr Gln Val Pro Glu Phe
           55                  60                  65
   gat tcg tcc aca att aaa aat atc tct tct gca aaa ggc ctg gac gtt       715
   Asp Ser Ser Thr Ile Lys Asn Ile Ser Ser Ala Lys Gly Leu Asp Val
       70                  75                  80
   tgg gac agc tgg cca tta caa aac gct gac ggc act gtc gca aac tat       763
   Trp Asp Ser Trp Pro Leu Gln Asn Ala Asp Gly Thr Val Ala Asn Tyr
   85                  90                  95                  100
   cac ggc tac cac atc gtc ttt gca tta gcc gga gat cct aaa aat gcg       811
   His Gly Tyr His Ile Val Phe Ala Leu Ala Gly Asp Pro Lys Asn Ala
                   105                 110                 115
   gat gac aca tcg att tac atg ttc tat caa aaa gtc ggc gaa act tct       859
   Asp Asp Thr Ser Ile Tyr Met Phe Tyr Gln Lys Val Gly Glu Thr Ser
               120                 125                 130
   att gac agc tgg aaa aac gct ggc cgc gtc ttt aaa gac agc gac aaa      907
   Ile Asp Ser Trp Lys Asn Ala Gly Arg Val Phe Lys Asp Ser Asp Lys
           135                 140                 145
   ttc gat gca aat gat tct atc cta aaa gac caa aca caa gaa tgg tca      955
   Phe Asp Ala Asn Asp Ser Ile Leu Lys Asp Gln Thr Gln Glu Trp Ser
       150                 155                 160
   ggt tca gcc aca ttt aca tct gac gga aaa atc cgt tta ttc tac act     1003
   Gly Ser Ala Thr Phe Thr Ser Asp Gly Lys Ile Arg Leu Phe Tyr Thr
   165                 170                 175                 180
   gat ttc tcc ggt aaa cat tac ggc aaa caa aca ctg aca act gca caa     1051
   Asp Phe Ser Gly Lys His Tyr Gly Lys Gln Thr Leu Thr Thr Ala Gln
                   185                 190                 195
   gtt aac gta tca gca tca gac agc tct ttg aac atc aac ggt gta gag     1099
   Val Asn Val Ser Ala Ser Asp Ser Ser Leu Asn Ile Asn Gly Val Glu
               200                 205                 210
   gat tat aaa tca atc ttt gac ggt gac gga aaa acg tat caa aat gta     1147
   Asp Tyr Lys Ser Ile Phe Asp Gly Asp Gly Lys Thr Tyr Gln Asn Val
           215                 220                 225
   cag cag ttc atc gat gaa ggc aac tac agc tca ggc gac aac cat acg     1195
   Gln Gln Phe Ile Asp Glu Gly Asn Tyr Ser Ser Gly Asp Asn His Thr
       230                 235                 240
   ctg aga gat cct cac tac gta gaa gat aaa ggc cac aaa tac tta gta     1243
   Leu Arg Asp Pro His Tyr Val Glu Asp Lys Gly His Lys Tyr Leu Val
   245                 250                 255                 260
   ttt gaa gca aac act gga act gaa gat ggc tac caa ggc gaa gaa tct     1291
   Phe Glu Ala Asn Thr Gly Thr Glu Asp Gly Tyr Gln Gly Glu Glu Ser
                   265                 270                 275
   tta ttt aac aaa gca tac tat ggc aaa agc aca tca ttc ttc cgt caa     1339
   Leu Phe Asn Lys Ala Tyr Tyr Gly Lys Ser Thr Ser Phe Phe Arg Gln
               280                 285                 290
   gaa agt caa aaa ctt ctg caa agc gat aaa aaa cgc acg gct gag tta     1387
   Glu Ser Gln Lys Leu Leu Gln Ser Asp Lys Lys Arg Thr Ala Glu Leu
           295                 300                 305
   gca aac ggc gct ctc ggt atg att gag cta aac gat gat tac aca ctg     1435
   Ala Asn Gly Ala Leu Gly Met Ile Glu Leu Asn Asp Asp Tyr Thr Leu
       310                 315                 320
   aaa aaa gtg atg aaa ccg ctg att gca tct aac aca gta aca gat gaa     1483
   Lys Lys Val Met Lys Pro Leu Ile Ala Ser Asn Thr Val Thr Asp Glu
   325                 330                 335                 340
   att gaa cgc gcg aac gtc ttt aaa atg aac ggc aaa tgg tac ctg ttc     1531
   Ile Glu Arg Ala Asn Val Phe Lys Met Asn Gly Lys Trp Tyr Leu Phe
                   345                 350                 355
   act gac tcc cgc gga tca aaa atg acg att gac ggc att acg tct aac    1579
   Thr Asp Ser Arg Gly Ser Lys Met Thr Ile Asp Gly Ile Thr Ser Asn
                   360                 365                 370
   gat att tac atg ctt ggt tat gtt tct aat tct tta act ggc cca tac    1627
   Asp Ile Tyr Met Leu Gly Tyr Val Ser Asn Ser Leu Thr Gly Pro Tyr
               375                 380                 385
   aag ccg ctg aac aaa act ggc ctt gtg tta aaa atg gat ctt gat cct    1675
   Lys Pro Leu Asn Lys Thr Gly Leu Val Leu Lys Met Asp Leu Asp Pro
           390                 395                 400
   aac gat gta acc ttt act tac tca cac ttc gct gta cct caa gcg aaa    1723
   Asn Asp Val Thr Phe Thr Tyr Ser His Phe Ala Val Pro Gln Ala Lys
   405                 410                 415                 420
   gga aac aat gtc gtg att aca agc tat atg aca aac aga gga ttc tac    1771
   Gly Asn Asn Val Val Ile Thr Ser Tyr Met Thr Asn Arg Gly Phe Tyr
                   425                 430                 435
   gca gac aaa caa tca acg ttt gcg cca agc ttc ctg ctg aac atc aaa    1819
   Ala Asp Lys Gln Ser Thr Phe Ala Pro Ser Phe Leu Leu Asn Ile Lys
                   440                 445                 450
   ggc aag aaa aca tct gtt gtc aaa gac agc atc ctt gaa caa gga caa    1867
   Gly Lys Lys Thr Ser Val Val Lys Asp Ser Ile Leu Glu Gln Gly Gln
               455                 460                 465
   tta aca gtt aac aaa taa aaacgcaaaa gaaaatgccg atatcctatt           1915
   Leu Thr Val Asn Lys
           470
   ggcattttct tttatttctt atcaacataa aggtgaatcc catatgaact atataaaagc  1975
   aggcaaatgg ctaaccgtat tcctaacctt ttgaagatc                         2014
<210> 8
   <211> 473
   <212> PRT
   <213> Bacillus subtilis
<400> 8
   Met Asn Ile Lys Lys Phe Ala Lys Gln Ala Thr Val Leu Thr Phe Thr
   1               5                   10                  15
   Thr Ala Leu Leu Ala Gly Gly Ala Thr Gln Ala Phe Ala Lys Glu Thr
               20                  25                  30
   Asn Gln Lys Pro Tyr Lys Glu Thr Tyr Gly Ile Ser His Ile Thr Arg
           35                  40                  45
   His Asp Met Leu Gln Ile Pro Glu Gln Gln Lys Asn Glu Lys Tyr Gln
       50                  55                  60
   Val Pro Glu Phe Asp Ser Ser Thr Ile Lys Asn Ile Ser Ser Ala Lys
   65                  70                  75                  80
   Gly Leu Asp Val Trp Asp Ser Trp Pro Leu Gln Asn Ala Asp Gly Thr
   85 90 95
   Val Ala Asn Tyr His Gly Tyr His Ile Val Phe Ala Leu Ala Gly Asp
   100 105 110
   Pro Lys Asn Ala Asp Asp Thr Ser Ile Tyr Met Phe Tyr Gln Lys Val
   115 120 125
   Gly Glu Thr Ser Ile Asp Ser Trp Lys Asn Ala Gly Arg Val Phe Lys
   130 135 140
   Asp Ser Asp Lys Phe Asp Ala Asn Asp Ser Ile Leu Lys Asp Gln Thr
   145 150 155 160
   Gln Glu Trp Ser Gly Ser Ala Thr Phe Thr Ser Asp Gly Lys Ile Arg
   165 170 175
   Leu Phe Tyr Thr Asp Phe Ser Gly Lys His Tyr Gly Lys Gln Thr Leu
   180 185 190
   Thr Thr Ala Gln Val Asn Val Ser Ala Ser Asp Ser Ser Leu Asn Ile
   195 200 205
   Asn Gly Val Glu Asp Tyr Lys Ser Ile Phe Asp Gly Asp Gly Lys Thr
   210 215 220
   Tyr Gln Asn Val Gln Gln Phe Ile Asp Glu Gly Asn Tyr Ser Ser Gly
   225 230 235 240
   Asp Asn His Thr Leu Arg Asp Pro His Tyr Val Glu Asp Lys Gly His
   245 250 255
   Lys Tyr Leu Val Phe Glu Ala Asn Thr Gly Thr Glu Asp Gly Tyr Gln
   260 265 270
   Gly Glu Glu Ser Leu Phe Asn Lys Ala Tyr Tyr Gly Lys Ser Thr Ser
   275 280 285
   Phe Phe Arg Gln Glu Ser Gln Lys Leu Leu Gln Ser Asp Lys Lys Arg
   290 295 300
   Thr Ala Glu Leu Ala Asn Gly Ala Leu Gly Met Ile Glu Leu Asn Asp
   305 310 315 320
   Asp Tyr Thr Leu Lys Lys Val Met Lys Pro Leu Ile Ala Ser Asn Thr
   325 330 335
   Val Thr Asp Glu Ile Glu Arg Ala Asn Val Phe Lys Met Asn Gly Lys
   340 345 350
   Trp Tyr Leu Phe Thr Asp Ser Arg Gly Ser Lys Met Thr Ile Asp Gly
   355 360 365
   Ile Thr Ser Asn Asp Ile Tyr Met Leu Gly Tyr Val Ser Asn Ser Leu
   370 375 380
   Thr Gly Pro Tyr Lys Pro Leu Asn Lys Thr Gly Leu Val Leu Lys Met
   385 390 395 400
   Asp Leu Asp Pro Asn Asp Val Thr Phe Thr Tyr Ser His Phe Ala Val
   405 410 415
   Pro Gln Ala Lys Gly Asn Asn Val Val Ile Thr Ser Tyr Met Thr Asn
   420 425 430
   Arg Gly Phe Tyr Ala Asp Lys Gln Ser Thr Phe Ala Pro Ser Phe Leu
   435 440 445
   Leu Asn Ile Lys Gly Lys Lys Thr Ser Val Val Lys Asp Ser Ile Leu
   450 455 460
   Glu Gln Gly Gln Leu Thr Val Asn Lys
   465 470
<210> 9
   <211> 20
   <212> DNA
   <213> Artificial
<220>
   <223> gluX primer 1
<400> 9
   ggcagcgatg aagcattgct 20
<210> 10
   <211> 40
   <212> DNA
   <213> Artificial
<220>
   <223> gluX primer 2
<400> 10
   agggcaatga agtcaagcag ctggacggat cgtggagatc 40
<210> 11
   <211> 40
   <212> DNA
   <213> Artificial
<220>
   <223> gluX primer 3
<400> 11
   gatctccacg atccgtccag ctgcttgact tcattgccct 40
<210> 12
   <211> 20
   <212> DNA
   <213> Artificial
<220>
   <223> gluX primer 4
<400> 12
   ttccgtttgc gctggtgttg 20
<210> 13
   <211> 40
   <212> DNA
   <213> Artificial
<220>
   <223> gluX primer 5
<400> 13
   gccgggatcc ttcccgctgc atccgcgagc tgtaccgcat 40
<210> 14
   <211> 40
   <212> DNA
   <213> Artificial
<220>
   <223> gluX primer 6
<400> 14
   gccgggatcc ttatccacag acccagccgc tattcgacaa 40
<210> 15
   <211> 21
   <212> DNA
   <213> Artificial
<220>
   <223> gluX primer 7
<400> 15
   gcttccggct cgtatgttgt g 21
<210> 16
   <211> 2283
   <212> DNA
   <213> Corynebacterium glutamicum
<220>
   <221> CDS
   <222> (1001)..(1279)
<400> 16
   gggtacttcg ccaggggtgg gggtgtattc cacacgagtt aacccaaggc gactttccgt     60
   attcctattc accaccacgc cgatcttcca cagattgccc agcatttcta cggcacgctg    120
   cgctttgccg ggtccggaaa actctttata tataaggttt ggttcggtga cagatccgag    180
   acgcctgcgc cacatatcct gattttcctg aaagtccggc gccacggcgt catcgcttcc    240
   cggctccaca tcggtgtgct gataaaaaat ctcaatctca gggatagcga tcattagtgg    300
   gtgcgcagtt tcagaaggct ggaacccaaa cgagcgccat tcagaatcgc tgcgcacatc    360
   gagcttgcca atcaggctgg tcagcgcgtc aaaggtgtca ggcagcgatg aagcattgct    420
   tgtcgacgca ccccccttgg ttcccgctgc atccgcgagc tgtaccgcat caagaagttg    480
   gatagccaac gatgcacctt gtttggtggt caagttgggt agtgcacgta ctgtttccac    540
   aatgaactgt tccacgcggc ttcgggctgc gggatcttgt tctttagaga tccgatcgat    600
   ggttgtttcg aaatcattca tggccggccc ctgtccttct taagcttgtc tctggtttct    660
   aagcataggc ataagcgcag ttcaataggg gaaatgacca ggaaacaagt tttattcaca    720
   ccctgggggt gattctagtc actaagttta gctaaggtgt cctgagttgc tttttgggta    780
   gcttaagtag ccttgacctg ctgttatgtt tttttgcggt ctggtataaa ttgtgccgat    840
   ttaaggattt tgtgggggtg gattgaaatt agttggcccg atcccactac ttttcgcctg    900
   gagtgcttgt aggttgatag aaagtaaact aaagtaaaca tcaggttaac agccgggggt    960
   tcaagtatta actccctcgg aaacagaaag gaacacgaca atg gct acc aca gct     1015
                                               Met Ala Thr Thr Ala
                                               1               5
   tcc aag atc tcc acg atc cgt cca gca cag caa gat gct ctt tgg agc     1063
   Ser Lys Ile Ser Thr Ile Arg Pro Ala Gln Gln Asp Ala Leu Trp Ser
                   10                  15                  20
   gta cgt gag gat ctt cac gct cgc ttc gat ggc ctg gtc gat cct gtc     1111
   Val Arg Glu Asp Leu His Ala Arg Phe Asp Gly Leu Val Asp Pro Val
                   25                  30                  35
   cag gta gac gca att ttg gac cat gtc gca tct aac cgc gaa gcc aag     1159
   Gln Val Asp Ala Ile Leu Asp His Val Ala Ser Asn Arg Glu Ala Lys
               40                  45                  50
   atc acc gtc ttc agc aag att ttc atc gct cgc gag gca acc gct gca     1207
   Ile Thr Val Phe Ser Lys Ile Phe Ile Ala Arg Glu Ala Thr Ala Ala
   55                  60                  65
   ctt cag cag att gct ggc aac gtt aac gca gac ctg ctt gac ttc att      1255
   Leu Gln Gln Ile Ala Gly Asn Val Asn Ala Asp Leu Leu Asp Phe Ile
   70                  75                  80                  85
   gcc ctc aac cgt ggc atg gca gca taagttttag ctgcccataa attaactaaa    1309
   Ala Leu Asn Arg Gly Met Ala Ala
                   90
   ggccgggatt cactcgatgt gaatcccggc ctttagtttt tagtcttgtg ctcagagttt  1369
   ccagatacag accaagcggc caataaagcc tattcggatt gtttcttagc cacctagttt  1429
   ggctattacg ccttggacac caaactaggc agccgaagac ttcccgaaac tcacctcatc  1489
   ggtcacatag gacggtcacg agggcactga gcaaacaaaa tagctgggat tcacatggtg  1549
   tgaatcccag ccagtcgcta ttttttaatc ctttttcgcg tctacttcgc cttcttcggt   1609
   cgattctggt gtccaaccat cggcccattt gccggtggtg aagtcataat caacatcatt  1669
   gccttcttcg tcggtgcgct gataccagtc ggtgacgtga tcagcggtgg aatcgaaggt  1729
   agcacccgct ccacgacctt cctctgcagg ttcgatggag agctcgaagt cgtcgccgtg  1789
   ttcttccaag ccacgaacca ccgcgttaga gatggctgcc tttgcatagc gttgtcgaat  1849
   agcggctggg tctgtggata aatccttaat aaaggcaata gccatcacga tcaacaccag  1909
   cgcaaacgga atggcgatca aaatggtgag gttctgcaga ccagtcagcg cggattcgcc  1969
   accagtaagc agcatgacca ccgcgatgcc catcatgcac agtccccaga acaccacgat  2029
   caatttgttt ggtgcagggt taccttggga gctcatcgtt cccatcacca cggaggcgga  2089
   atcggcagag gtaacaaaga atactgccag cacaaagatc aaaatgaacg gtgtgatcga  2149
   gtacagcgga aggttgctga acatatcaaa cagcacctgt tccttggatg aactgccgtc  2209
   aaaaccatct acgttctcgc ggttcatcgt gatggcagtt ccaccgaaaa tggtgaacgc  2269
   caggatcaaa atga                                                     2283
<210> 17
   <211> 93
   <212> PRT
   <213> Corynebacterium glutamicum
<400> 17
   Met Ala Thr Thr Ala Ser Lys Ile Ser Thr Ile Arg Pro Ala Gln Gln
   1               5                   10                  15
   Asp Ala Leu Trp Ser Val Arg Glu Asp Leu His Ala Arg Phe Asp Gly
               20                  25                  30
   Leu Val Asp Pro Val Gln Val Asp Ala Ile Leu Asp His Val Ala Ser
           35                  40                  45
   Asn Arg Glu Ala Lys Ile Thr Val Phe Ser Lys Ile Phe Ile Ala Arg
       50                  55                  60
   Glu Ala Thr Ala Ala Leu Gln Gln Ile Ala Gly Asn Val Asn Ala Asp
   65                  70                  75                  80
   Leu Leu Asp Phe Ile Ala Leu Asn Arg Gly Met Ala Ala
                   85                  90

## Description

### Technical Field

The present invention relates to fermentation industry, and it relates to a method for efficiently producing L-glutamic acid using a coryneform bacterium.

### Background Art

L-glutamic acid is conventionally produced by fermentation using coryneform bacteria belonging to the genus *Brevibacterium, Corynebacterium* or the like having an L-glutamic acid producing ability. In order to improve productivity of these coryneform bacteria, bacterial strains isolated from the nature, or artificial mutant strains or strains modified by gene recombination of them are used.

As coryneform bacteria of which L-glutamic acid producing ability is improved by gene recombination techniques, to date developed are coryneform bacteria of which glutamate dehydrogenase activity, citrate synthase activity and pyruvate carboxylase activity are enhanced (Patent document 1), coryneform bacteria of which α-ketoglutarate dehydrogenase activity, or α-ketoglutarate dehydrogenase and isocitrate lyase activities are reduced (Patent documents 2 and 3), and so forth.

Meanwhile, the entire nucleotide sequence of *Corynebacterium glutamicum* chromosome have already been determined (Non-patent document 1). However, about 40% of 3099 of putative orfs thereof show low homology to genes of other microorganisms of which functions are known, and encode proteins of which functions are unknown, and influence of deletion of these genes has not been known to date.
Patent document 1: International Patent Publication WO00/18935
Patent document 2: International Patent Publication WO95/34672
Patent document 3: Japanese Patent Laid-open (KOKAI, JP-A) No. 01-296994
Non-patent document 1: Appl. Microbiol. Biotechnol., 62 (2-3), pp.99-109 (2003)

### Disclosure of the Invention

An object of the present invention is to provide a coryneform bacterium of which L-glutamic acid producing ability is improved, and to provide a method for efficiently producing L-glutamic acid using such a bacterium.

The inventors of the present invention conducted various researches in order to achieve the aforementioned object. As a result, they found that if the *gluX* gene was inactivated in coryneform bacteria, L-glutamic acid producing ability thereof could be improved, and thus accomplished the present invention.

That is, the present invention provides the followings.
(1) A method for producing L-glutamic acid, which comprises culturing a corneyform bacterium having L-glutamic acid producing ability in a medium to produce and accumulate L-glutamic acid, and collecting L-glutamic acid from the medium, wherein said coryneform bacterium is modified so that the *gluX* gene on the chromosome is disrupted and
   wherein said *gluX* gene is selected from the group consisting of:
   (a) a DNA which comprises the nucleotide sequence consisting of 1001 to 1279 of SEQ ID NO: 16, and
   (b) a DNA which is able to hybridize with the nucleotide sequence consisting of 1001 to 1279 of SEQ ID NO: 16 under stringent conditions comprising washing at 0.1 x SSC, 0.1% SDS at 60° C, and encodes a protein having a function of improving production amount of L-glutamic acid when the DNA is inactivated on a chromosome.
(2) The method according to (1), wherein said *gluX* gene encodes a protein selected from the group consisting of:
   (A) a protein comprising the amino acid sequence of SEQ ID NO: 17, and
   (B) a protein comprising the amino acid sequence of SEQ ID NO: 17 including substitution, deletion, insertion, or addition of one to 10 amino acid residues and having a function of improving production amount of L-glutamic acid when the DNA is inactivated on a chromosome.
(3) The method according to according to (1) or (2), wherein said corneyform bacterium is *Corneybacterium glutamicum.*

### Brief Explanation of the Drawings

Fig. 1: A drawing showing the construction procedure of the plasmid pBS3.
Fig. 2: A drawing showing the construction procedure of the plasmid pBS4S.
Fig. 3]: A drawing showing the construction procedure of the plasmid pBXGXD for disruption of *gluX.*

### Description of the Preferred Embodiments

Hereafter, embodiments of the present invention will be explained in detail.

### <1> Coryneform bacterium of the present invention

The coryneform bacteria used for the present invention include *Corynebacterium* bacteria and those bacteria having been previously classified into the genus *Brevibacterium* but have been re-classified into the genus *Corynebacterium* (Int. J. Syst. Bacteriol., 41, 255 (1981)), and further include bacteria belonging to the genus *Brevibacterium,* which is extremely close to the genus *Corynebacterium.* Specific examples include the followings.:
*Corynebacterium acetoacidophilum*
*Corynebacterium acetoglutamicum*
*Corynebacterium alkanolyticum*
*Corynebacterium callunae*
*Corynebacterium glutamicum*
*Corynebacterium lilium*
*Corynebacterium melassecola*
*Corynebacterium thermoaminogenes (Corynebacterium efficiens)*
*Corynebacterium herculis*
*Brevibacterium divaricatum*
*Brevibacterium flavum*
*Brevibacterium immariophilum*
*Brevibacterium lactofermentum (Corynebacterium glutamicum)*
*Brevibacterium roseum*
*Brevibacterium saccharolyticum*
*Brevibacterium thiogenitalis*
*Brevibacterium ammoniagenes*
*Brevibacterium album*
*Brevibacterium cerinum*
*Microbacterium ammoniaphilum*

Specifically, the following strains can be encompassed:
*Corynebacterium acetoacidophilum* ATCC 13870
*Corynebacterium acetoglutamicum ATCC* 15806
*Corynebacterium alkanolyticum* ATCC 21511
*Corynebacterium callunae* ATCC 15991
*Corynebacterium glutamicum* ATCC 13020, ATCC 13032, ATCC 13060, ATCC 13869
*Corynebacterium lilium* ATCC 15990
*Corynebacterium melassecola* ATCC 17965
*Corynebacterium thermoaminogenes* AJ12340 (FERM BP-1539)
*Corynebacterium herculis* ATCC 13868
*Brevibacterium divaricatum* ATCC 14020
*Brevibacterium flavum* ATCC 13826, ATCC 14067, AJ12418 (FERM BP-2205)
*Brevibacterium immariophilum* ATCC 14068
*Brevibacterium lactofermentum (Corynebacterium glutamicum)* ATCC 13869
*Brevibacterium roseum* ATCC 13825
*Brevibacterium saccharolyticum* ATCC 14066
*Brevibacterium thiogenitalis* ATCC 19240
*Brevibacterium ammoniagenes* ATCC 6871, ATCC 6872
*Brevibacterium album* ATCC 15111
*Brevibacterium cerinum* ATCC 15112
*Microbacterium ammoniaphilum* ATCC 15354

These strains are available from the American Type Culture Collection. That is, each strain is given a registration number. Strains can be ordered using this registration number. The registration number corresponding to each strain is listed in the catalogue of the ATCC. The AJ 12340 strain was deposited at National Institute of Bioscience and Human Technology, Agency of Industrial Science and Technology, Ministry of International Trade and Industry (currently International Patent Organism Depositary, National Institute of Advanced Industrial Science and Technology at Tsukuba Central 6, 1-1, Higashi 1-chome, Tsukuba-shi, Ibaraki-ken 305-5466, Japan) on October 27, 1987 under the provisions of the Budapest Treaty and given an accession number of FERM BP-1539. The AJ12418 strain was deposited at National Institute of Bioscience and Human Technology, Agency of Industrial Science and Technology, Ministry of International Trade and Industry on January 5, 1989 under the provisions of the Budapest Treaty and given an accession number of FERM BP-2205.

In the present invention, "L-glutamic acid producing ability" means an ability to accumulate L-glutamic acid in a medium when the coryneform bacterium of the present invention is cultured in the medium. The bacterium of the present invention may have this L-glutamic acid producing ability as a property of a wild strain of the coryneform bacterium, or the ability may be a property imparted by breeding. Furthermore, the L-glutamic acid producing ability may be imparted by modifying the bacterium so that the *gluX is* inactivated in the manner described later.

In order to impart L-glutamic acid producing ability by breeding, there can be used methods conventionally used for breeding of coryneform bacteria, for example, those used for acquisition of metabolic regulation mutant strains, construction of recombinant strains in which an enzyme of biosynthesis system of an objective substance is enhanced (refer to "Amino Acid Fermentation", the Japan Scientific Societies Press [Gakkai Shuppan Center], 1st Edition, published on May 30, 1986, pp.77-100) and so forth. In those methods, the metabolic regulation mutation to be imparted, or enhancement of biosynthesis system enzymes for an objective substance and so forth may be individually imparted or performed, or two or more of them may be imparted in combination. The impartation of metabolic regulation mutation and the enhancement of a biosynthesis system enzyme may be combined.

Hereinafter, methods for imparting L-glutamic acid producing ability are described. Examples of the method for imparting L-glutamic acid-producing ability by breeding include enhancing expression of a gene encoding an enzyme involved in L-glutamic acid biosynthesis. Examples of the enzyme involved in L-glutamic acid biosynthesis include glutamate dehydrogenase, glutamine synthetase, glutamate synthase, isocitrate dehydrogenase, aconitate hydratase, citrate synthase, phosphoenolpyruvate carboxylase, pyruvate carboxylase, pyruvate dehydrogenase, pyruvate kinase, phosphoenolpyruvate synthase, enolase, phosphoglyceromutase; phosphoglycerate kinase, glyceraldehyde-3-phophate dehydrogenase, triose phosphate isomerase, fructose bisphosphate aldolase, phosphofructokinase, glucose phosphate isomerase, and so forth.

Enhancing expression of these genes can be achieved by introducing an amplification plasmid obtained by introducing a DNA fragment containing any of these genes into a suitable plasmid, for example, a plasmid vector containing at least a gene responsible for replication of the plasmid in coryneform bacteria, by increasing copy number of these genes in a chromosome by conjugation, gene transfer etc., by introducing a mutation into a promoter region of these genes, or by replacing a promoter with a stronger promoter (refer to International Patent Publication WO00/18935).

When a gene is amplified in a plasmid or the chromosome, the promoter for expression of the gene may be any promoter so long as a promoter that functions in coryneform bacteria is chosen, and it may be an inherent promoter of the gene used. Expression amount of the genes can also be controlled by suitably choosing the promoter. Examples of the coryneform bacteria modified so that expression of citrate synthase gene, phosphoenolpyruvate carboxylase gene, glutamate dehydrogenase gene, and/or isocitrate dehydrogenase gene is enhanced include the microorganisms described in International Patent Publication WO00/18935 and European Patent Publication No. 1010755.

Moreover, the modification for imparting the L-glutamic acid producing ability may also be attained by reducing or deleting activity of an enzyme that catalyzes a reaction branching from a biosynthetic pathway of L-glutamic acid to produce another compound. Examples of enzymes that catalyze a reaction to synthesize a compound other than L-glutamic acid include isocitrate lyase, α-ketoglutarate dehydrogenase, acetyl phosphate transferase, acetate kinase, acetohydroxy acid synthase, acetolactate synthase, acetyl formate transferase, lactate dehydrogenase, glutamate decarboxylase, 1-pyrroline dehydrogenase, and so forth.

In order to reduce or delete activity of any one of the enzymes described above, a mutation that reduces or deletes the intracellular activity of the enzyme can be introduced into the gene of the aforementioned enzyme on a chromosome by a usual mutagenesis method. For example, it can be attained by deleting the gene encoding the enzyme on a chromosome, or modifying an expression control sequence such as promoter, Shine-Dalgarno (SD) sequence, operator, terminator and attenuator by gene recombination. Furthermore, it can also be attained by introducing a mutation for amino acid substitution (missense mutation), a stop codon (nonsense mutation), or a frame shift mutation that adds or deletes one or two nucleotides into a region encoding the enzyme on a chromosome, or by deleting the gene partially or entirely (Journal of Biological Chemistry, 272:8611-8617 (1997)). Furthermore, the enzymatic activity can also be reduced or deleted by constructing a gene encoding a mutant enzyme of which coding region is deleted, and substituting the constructed gene for the normal gene on a chromosome by homologous recombination or the like. Examples of coryneform bacteria of which α-ketoglutarate dehydrogenase activity is reduced include the following strains described in Japanese Patent Laid-open Nos. 07-834672, 06-237779, 1-296994, etc.
*Brevibacterium lactofermeraum* ΔS strain (WO95/34672)
*Brevibacterium lactofermentum* AJ12821 strain (FERM BP-4172; see FR9401748)
*Brevibacteriumflavum* AJ12822 strain (FERM BP-4173; see FR9401748)
*Corynebacterium glutamicum* AJ12823 strain (FERM BF-4174; see FR9401748)

Other methods of imparting the L-glutamic acid producing ability include imparting resistance to organic acid analogs or respiratory inhibitors, or sensitivity to inhibitor of cell wall synthesis. Examples of such methods include, for example, imparting resistance to benzopirone or naphtoquinones (JP56-1889A), imparting resistance to HOQNO (JP56-140895A), imparting resistance to α-ketomalonic acid (JP57-2689A), imparting resistance to guanidine (JP56-35981A), imparting sensitivity to penicillin (JP04-88994A), and the like.

Specific examples of such bacteria include the following strains:
*Brevibacterium flavum* AJ11355 (FERM P-5007; JP56-1889A)
*Corynebacterium glutamicum* AJ11368 (FERM P-5020; JP56-1889A)
*Brevibacterium flavum* AJ11217 (FERM P-4318; JP57-2689A)
*Corynebacterium glutamicum* AJ11218 (FERM P-4319; JP57-2689A)
*Brevibacterium flavum* AJ11564 (FERM P-5472; JP56-140895A)
*Brevibacterium flavum* AJ11439 (FERM P-5136; JP56-35981A)
*Corynebacterium glutamicum* H7684 (FERM BP-3004; JP04-88994A)

The parent strain from which the coryneform bacterium of the present invention is obtained may also be a coryneform bacterium which produces L-glutamic acid under conditions that induce L-glutamic acid production, such as biotin restricted condition, surfactant added condition and penicillin added condition (henceforth referred to as "L-glutamic acid producing conditions" for convenience). The "L-glutamic acid producing conditions" means conditions that a substance that induces L-glutamic acid production is added to a medium which contains a carbon source, a nitrogen source, inorganic salts, and a trace amount of organic nutrients, such as amino acids and vitamins, if necessary, and conditions that the amount of a substance that inhibits the L-glutamic acid production is limited in a medium. The substances that induce L-glutamic acid production include antibiotics such as penicillin G and surfactants including saturated fatty acid, such as Tween 40, 60, or the like. The substance that inhibits the L-glutamic acid production includes biotin (Amino Acid Fermentation, Japan Scientific Societies Press 1986).

Herein, concentration of the above substances in a medium under L-glutamic acid producing conditions are as follows. The concentration of biotin is less than 30 µg/L, preferably less than 20 µg/L, more preferably less than 10 µg/L, and the medium may not contain biotin at all. The concentration of penicillin in the medium is not less than 0.1 U/ml, preferably not less than 0.2 U/ml, more preferably not less than 0.4 U/ml. The concentration of surfactant in the medium is not less than 0.5 g/L, preferably not less than 1 g/L, more preferably not less than 2 g/L. However, any concentration of these substances can be applied so long as L-glutamic acid production is induced. In addition, when a medium contains antibiotic or surfactant, it is preferable that the medium contains high concentration of biotin. In such a case, it is preferable that the medium contains more than 50 µg/L, preferably more than 100 µg/L, more preferably more than 200 µg/L, of biotin.

Examples of the parent strain used in the present invention include wild type strains of coryneform bacteria described above, or the following strains:
*Brevibacterium flavum* AJ11217 (FERM P-4318; JP57-2689A)
*Corynebacterium glutamicum* AJ11218 (FERM P-4319; JP57-2689A)
*Brevibacterium lactofermentum* AJ11426 (FERM P-5123 JP56-048890A)
*Corynebacterium glutamicum* AJ11440 (FERM P-5137 JP56-048890A)
*Brevibacterium lactofermentum* AJ11796 (FERM P-6402 JP58-158192A)

The coryneform bacterium of the present invention is a coryneform bacterium which has the aforementioned L-glutamic acid producing ability, and is modified so that the *gluX* gene is inactivated. The coryneform bacterium of the present invention can be obtained by modifying a coryneform bacterium having L-glutamic acid producing ability so that the *gluX* gene is inactivated. In the breeding of the coryneform bacterium of the present invention, either the impartation of the L-glutamic acid producing ability or the modification for inactivating the *gluX* gene may be performed first.

The expression "being modified so that the *gluX* gene is inactivated" corresponds to a state that the number of the molecules of the GluX protein encoded by the *gluX* gene per cell is reduced compared with that of the parent strain or a wild type strain, a state that the activity of GluX protein per molecule is reduced, a state that the GluX protein molecule is no longer produced at all, and so forth. For example, the expression means that amount of the protein encoded by the *gluX* gene is reduced compared with that of a non-modified strain or a wild strain, that expression amount of the *gluX* gene is reduced, that three-dimensional conformation of the protein is modified, and thus the normal GluX protein cannot be produced, or that the GluX protein that can be produced by the wild type strain or non-modified strain of the coryneform bacterium cannot be produced at all. The wild-type coryneform bacterium used as the reference for the comparison is, for example, *Corynebacterium glutamicum (Brevibacterium lactofermentum)* ATCC 13869 or ATCC 13032.

Reduction of expression amount of the *gluX* gene can be confirmed by comparing amount of mRNA of the *gluX* with that of a wild type or non-modified strain. Examples of the method for confirming the expression amount include Northern hybridization and RT-PCR (Molecular Cloning, Cold Spring Harbor Laboratory Press, Cold Spring Harbor (USA) 2001). Reduction degree of the expression amount is not particularly limited so long as it is reduced compared with that of a wild type strain or non-modified strain. However, it is desirably reduced, for example, by at least 75%, 50%, 25%, or 10% or less compared with a wild strain or non-modified strain, or the expression may be completely eliminated.

Reduction of the amount of the protein encoded by the *gluX* gene can be confirmed by detection based on Western blotting using antibodies (Molecular Cloning, Cold Spring Harbor Laboratory Press, Cold Spring Harbor (USA), 2001). Reduction degree of the production amount is not particularly limited so long as it is reduced compared with that of a wild strain or non-modified strain. However, it is desirably reduced, for example, by at least 75%, 50%, 25%, or 10% or less compared with a wild strain or non-modified strain, or the activity may be completely eliminated, which means that the protein is not produced at all.

The protein referred to in the present invention encoded by the *gluX* gene (GluX protein) is a protein having a function of improving production amount of L-glutamic acid through inactivation of the *gluX* gene on a chromosome. Here, examples of the GluX protein of coryneform bacteria include the protein (SEQ ID NO: 17) encoded by the *gluX* gene of *Corynebacterium glutamicum (Brevibacterium lactofermentum)* ATCC 13869 or *Corynebacterium glutamicum* ATCC 13032 (nucleotide numbers 1001 to 1279 of SEQ ID NO: 16). The *gluX* gene of *Corynebacterium glutamicum* ATCC 13032 is encoded by the nucleotides 2475838 to 2476119 in the genome sequence registered as Genbank Accession No. NC_003450, and it is registered as NCg12252. Moreover, since the nucleotide sequence of the *gluX* gene may differ depending on species or strains of coryneform bacteria, the *gluX* gene may be a variant of the nucleotide sequence of the nucleotide numbers 1001 to 1279 of SEQ ID NO: 16. Variants of the *gluX* gene can be searched for with reference to the sequence of the nucleotide sequence of nucleotide numbers 1001 to 1279 of SEQ ID NO: 16 using BLAST or the like (http://blast.genome.jp/). Moreover, variants of the *gluX* gene include *gluX* gene homologues, for example, genes that can be amplified by PCR using a chromosome of a coryneform bacterium as a template and synthetic oligonucleotides of SEQ ID NOS: 13 and 14.

Examples of the GluX protein include those having the amino acid sequence of SEQ ID NO: 17. However, since the codons used may differ depending on species or strains of coryneform bacteria, and the nucleotide sequence of the gene encoding GluX may differ, *gluX* may encode such amino acid. sequences as described above including substitution, deletion, insertion, or addition of one or several amino acid residues so long as the function of the GluX protein does not change. Here, number meant by the term "several" is, for example, 2 to 20, preferably 2 to 10, more preferably 2 to 5. The aforementioned substitution, deletion, insertion, or addition of one or several amino acid residues is a conservative mutation that allows normal production of the GluX protein. Conservative substitutions include: substitutions of Phe, Trp, and Tyr for each other in the case of aromatic amino acids, substitutions of Leu, Ile, and Val for each other in the case of hydrophobic amino acids, substitutions of Gln and Asn for each other in the case of polar amino acids, substitutions of Arg, Lys, and His for each other in the case of basic amino acids, substitutions of Asp and Glu for each other in the case of acidic amino acids, and substitutions of Ser and Thr for each other in the case of hydroxyl group-containing amino acids. Typical conservative mutations are conservative substitutions, which include: substitution of Ser or Thr for Ala, substitution of Gin, His, or Lys for Arg, substitution of Glu, Gln, Lys, His, or Asp for Asn, substitution of Asn, Glu, or Gln for Asp, substitution of Ser or Ala for Cys, substitution of Asn, Glu, Lys, His, Asp, or Arg for Gln, substitution of zGly, Asn, Gln, Lys, or Asp for Glu, substitution of Pro for Gly, substitution of Asn, Lys, Gln, Arg, or Tyr for His, substitution of Leu, Met, Val, or Phe for Ile, substitution of Ile, Met, Val, or Phe for Leu, substitution of Asn, Glu, Gln, His, or Arg for Lys, substitution of Ile, Leu, Val, or Phe for Met, substitution of Trp, Tyr, Met, Ile, or Leu for Phe, substitution of Thr or Ala for Ser, substitution of Ser or Ala for Thr, substitution of Phe or Tyr for Trp, substitution of His, Phe, or Trp for Tyr and substitution of Met, Ile, or Leu for Val.

A variant of the *gluX* gene may also be a DNA which is able to hybridize with the nucleotide sequence consisting of 1001 to 1279 of SEQ ID NO: 16, or a probe that can be prepared from the nucleotide sequence under stringent conditions. The "stringent conditions" are conditions under which a so-called specific hybrid is formed, and a nonspecific hybrid is not formed. Examples of the stringent conditions include those under which highly homologous DNAs hybridize to each other, for example, DNAs not less than 80, 90, 95, or 97% homologous, hybridize to each other, and DNAs less homologous than the above do not hybridize to each other, or conditions of washing once or preferably 2 or 3 times at a salt concentration and temperature corresponding to washing of typical Southern hybridization, i.e., 1 x SSC, 0.1% SDS at 60°C, preferably 0.1 x SSC, 0.1% SDS at 60°C, more preferably 0.1 x SSC,0.1% SDS at 68°C. Length of the probe may be arbitrarily selected depending on hybridization conditions. But, the length is usually 100 bps to 1 kbps.

' The expression "modified so that the *gluX* gene is inactivated" means that the modification is performed so that the GluX protein does not function normally. Bacteria modified in such a manner can be obtained by breeding a bacterium in which a mutation is introduced into the GluX protein using a reagent or the like so that the protein does not function normally, a bacterium in which a mutation is introduced into the *gluX* gene by gene engineering techniques or the like so that the amount of the GluX protein is reduced, or the bacterium no longer produces the GluX protein, or the like.

This may be achieved by, for example, deleting the *gluX* gene on the chromosome, or by modifying an expression control sequence such as a promoter, Shine Dargamo (SD) sequence, or the like. In addition, the modification may also be achieved by introducing an amino acid substitution (missense mutation), a stop codon (nonsense mutation), or a frame shift mutation which adds or deletes one or two nucleotides into a coding region, or by deleting a portion or the entire gene (Journal of biological Chemistry 272:8611-8617 (1997), Proceedings of the National Academy of Sciences,USA 95 5511-5515(1998), Journal of Biological Chemistry 266,20833-20839(1991)).

As the region to be deleted, either a region on the N-terminus side or a region on the C-terminus side may be deleted so long as a GluX protein which does not function normally is produced. Furthermore, inactivation of the *gluX* gene can also be attained by introducing a transposon carrying an antibiotic resistance gene or a gene useful for L-glutamic acid production into the coding region of *gluX.*

Introduction of such mutations as described above into the *gluX* gene can be attained by, for example, preparing a deletion type gene obtained by such modification that the gene includes deletion of a partial sequence of *gluX,* and does not produce the GluX protein which functions normally, and transforming a coryneform bacterium with a DNA containing the gene to cause homologous recombination of the deletion type gene and the gene on a chromosome and thereby disrupt the *gluX* gene on a chromosome. Such gene disruption utilizing gene substitution based on homologous recombination has already been established, and examples of the method therefor include the methods using a linear DNA called "Red-driven integration" developed by Datsenko and Wanner (Proc. Natl. Acad. Sci. USA, 2000, Vol. 97, No. 12, pp.6640-6645),or a methods using a plasmid containing a temperature sensitive replication origin (U.S. Patent No. 6,303,383, Japanese Patent Laid-open No. 05-007491) and so forth. Moreover, such gene disruption based on gene substitution utilizing homologous recombination as described above can also be performed using a plasmid not showing a replication ability in a host, or a plasmid which is capable of conjugative transfer to coryneforrn bacteria.

Examples of the temperature sensitive plasmid for coryneform bacterium include p48K and pSFKT2 (see Japanese Patent Laid-open No. 2000-262288 for these), pHSC4 (see French Patent Laid-open No. 2667875, 1992 and Japanese Patent Laid-open No. 5-7491) and so forth. These plasmids can autonomously replicate at 25°C at least, but cannot autonomously replicate at 37°C in coryneform bacteria. The *Escherichia coli* AJ12571 harboring pHSC4 was deposited at the National Institute of Bioscience and Human-Technology, the Agency of Industrial Science and Technology, the Ministry of International Trade and Industry (currently, the independent administrative corporation, National Institute of Advanced Industrial Science and Technology, International Patent Organism Depositary (Tsukuba Central 6, 1-1, Higashi 1-Chome, Tsukuba-shi, Ibaraki-ken, Japan, postal code: 305-5466)) on October 11,1990, receiving an accession number of FERM P-11763. Then, the deposit was converted into an international deposit under the provisions of the Budapest Treaty on August 26, 1991, receiving an accession number of FERM BP-3524.

As the plasmid which does not replicate in coryneform bacteria, a plasmid which is replicable in *Escherichia coli* is preferred, and examples include pHSG299 (Takara Bio Inc.), pHSG399 (Takara Bio Inc.), and so forth. Examples of the plasmid capable of conjugative transfer include pK19mobsacB (J. Bacteriology, 174:5462-65 (1992)). Examples of the deletion type gene modified so that it does not produce the GluX protein include the gene including deletion of the entire or partial region of SEQ ID NO: 16, the gene introduced with a missense mutation, the gene inserted with a transposon or a marker gene, the gene introduced with a nonsense mutation, and the gene introduced with a frameshift mutation, but the examples are not limited to these.

The inactivation of the *gluX* gene may be performed by the following methods. The deletion type gene of the *gluX* gene can be obtained by the following methods. The *gluX* gene can be obtained by preparing a chromosomal DNA from a coryneform bacterium by the method of Saito and Miura (refer to H. Saito and K. Miura, Biochem. Biophys. Acta, 72, 619 (1963); Text for Bioengineering Experiments, Edited by the Society for Bioscience and Bioengineering, Japan, pp.97-98, Baifukan, 1992), and performing PCR with it using oligonucleotides constructed based on sequences of known database such as Genbank, and the gene can be cloned using synthetic oligonucleotides of SEQ ID NOS: 13 and 14. The deletion type gene can be obtained by amplifying the full length of the *gluX* gene by PCR, and digesting the PCR product with a restriction enzyme which cleaves an internal site, or by amplifying a part of the coding region of the *gluX* gene by PCR.

Then, the deletion type gene is introduced into a plasmid which is temperature sensitive in coryneform bacteria, or a plasmid which cannot replicate in coryneform bacteria, and a coryneform bacterium is transformed with the recombinant plasmid. Transformation may be performed according to a method which has been reported to date. Examples of the transformation method include treating recipient cells with calcium chloride so as to increase permeability for the DNA, which has been reported for *Escherichia coli* K-12 (Mandel, M. and Higa, A., J. Mol. Biol., 53, 159 (1970)), and preparing competent cells from cells which are at the growth phase, followed by transformation with DNA, which has been reported for *Bacillus subtilis* (Duncan, C.H., Wilson, GA. and Young, F.E., Gene, 1, 153 (1977)). Alternatively, introducing a recombinant DNA into recipient cells, which have been reported to be applicable to *Bacillus subtilis, actinomycetes,* and yeasts (Chang, S. and Choen, S.N., Molec. Gen. Genet., 168, 111 (1979); Bibb, M.J., Ward, J.M. and Hopwood, O.A., Nature, 274, 398 (1978); Hinnen, A., Hicks, J.B. and Fink, G.R., Proc. Natl. Sci., USA, 75, 1929 (1978)), can be employed. In addition, transformation of coryneform bacteria can also be performed by the electric pulse method (JP2-207791A). When the transformation is performed with a plasmid which does not replicate in coryneform bacteria, the *gluX* gene on the plasmid and the *gluX* gene on a chromosome are recombined in the transformant obtained as described above. When a temperature sensitive plasmid is used, the transformant is cultured at a temperature at which the temperature sensitive replication origin does not function (25°C) to obtain a strain introduced with the plasmid. The strain introduced with the plasmid is cultured at a high temperature to eliminate the temperature sensitive plasmid, and this strain is applied to a plate containing an antibiotic. Since the temperature sensitive plasmid cannot proliferate at the high temperature, the strain of which plasmid is eliminated cannot grow on the plate containing an antibiotic, but a strain in which the *gluX* gene on the plasmid and the *gluX* gene on a chromosome are recombined appears, although it appears at an extremely low frequency.

In the strain introduced with the recombinant DNA into a chromosome as described above, recombination with the *gluX* gene sequence originally existing on the chromosome is caused, and thus two of fusion genes of the chromosomal *gluX* gene and the deletion type *gluX* gene, and the other portion of the recombinant DNA (vector portion, temperature sensitive replication origin, and drug resistance marker) between the fusion genes are inserted in the chromosome.

Then, in order to leave only the deletion type *gluX* gene on the chromosomal DNA, the *gluX* gene is eliminated together with the vector portion (including temperature sensitive replication origin and drug resistance marker) from the chromosomal DNA. In this case, the normal *gluX* gene is left on the chromosomal DNA, and the deletion type *gluX* gene is excised, or to the contrary, the deletion type *gluX* gene is left on the chromosomal DNA, and the normal *gluX* gene is excised. By selecting a strain in which the deletion type *gluX* gene is left on the chromosome by PCR or Southern hybridization, a strain in which the *gluX* gene is disrupted can be obtained.

Furthermore, the *sacB* gene encoding the levansucrase, which fatally functions in coryneform bacteria, may also be used as a marker of the homologous recombination (Schafer, A. et al., Gene, 145, pp.69-73 (1994)). That is, if levansucrase is expressed in coryneform bacteria, levan produced by assimilation of sucrose fatally functions, and therefore they cannot grow. Therefore, if a strain in which a vector encoding levansucrase remains on a chromosome is cultured on a plate containing sucrose, the strain cannot grow, and thus only a strain in which the vector is eliminated can be selected on a plate containing sucrose.

As the *sacB* gene or homologous gene thereof, the following sequences can be used.
*Bacillus subtilis: sacB* GenBank Accession Number X02730 (SEQ ID NO: 7)
*Bacillus amyloliquefaciens: sacB* GenBank Accession Number X52988
*Zymomonas mobilis:* sacB GenBankAccession Number L33402
*Bacillus stearothermophilus: surB* GenBank Accession Number U34874
*Lactobacillus sanfranciscensis: frfA GenBank* Accession Number AJ508391
*Acetobacter xylinus: lsxA* GenBank Accession Number AB034152
*Gluconacetobacter diazotrophicus: lsdA* GenBank Accession Number L41732

Inactivation of *gluX* can also be attained by, in addition to the aforementioned deletion of the gene encoding *gluX* on a chromosome, introduction of a mutation which inactivates *gluX* by modifying an expression control sequence such as promoter, Shine-Dalgarno (SD) sequence, operator, terminator and attenuator, or the like. An expression control sequence on a chromosome can be confirmed using gene analysis software such as Genetix, or a vector for expression analysis such as a promoter probe vector, or on the basis of known information such as that obtained from Genbank or the like. The mutation which inactivates *gluX* is, for example, a mutation which replaces the promoter region of *gluX* with a weaker promoter, or a mutation which makes the promoter away from a consensus sequence. Introduction of these mutations can be attained using a temperature sensitive plasmid or a plasmid which does not have replication ability as in the case mentioned above.

In addition to the aforementioned gene manipulation methods, examples of the method for inactivating *gluX* include, for example, treating a coryneform bacterium with ultraviolet irradiation or a mutagen used for usual mutagenesis such as N-methyl-N'-nitro-N-nitrosoguanidine (NTG) or nitrous acid, and selecting a strain in which *gluX* is inactivated. Moreover, inactivated *gluX* gene can also be obtained by artificially introducing a mutation into *gluX* by gene recombination based on error-prone PCR, DNA shuffling or StEP-PCR (Firth AE, Patrick WM, Bioinformatics, 2005 Jun 2, Statistics of protein library construction)

### <3> Production of L-glutamic acid using coryneform bacterium of the present invention

L-glutamic acid can be efficiently produced by culturing a coryneform bacterium obtained as described above in a medium to produce and accumulate L-glutamic acid in the medium and collecting L-glutamic acid from the medium.

In order to produce L-glutamic acid using the coryneform bacterium of the present invention, culture may be performed by a conventional method using an ordinary medium that contains a carbon source, a nitrogen source, an inorganic salt, and optionally organic micronutrients such as amino acids and vitamins. Either a synthetic medium or a natural medium may be used. Any kinds of carbon and nitrogen sources may be used so long as they can be utilized by the strain being cultured.

Saccharides such as glucose, glycerol, fructose, sucrose, maltose, mannose, galactose, starch hydrolysate, molasses, and so forth may be used as the carbon source. In addition, organic acids such as acetic acid and citric acid, and alcohols such as ethanol may also be used alone or in combination with other carbon sources.

Ammonia, ammonium salts such as ammonium sulfate, ammonium carbonate, ammonium chloride, ammonium phosphate, and ammonium acetate, nitrates, and so forth may be used as the nitrogen source.

Amino acids, vitamins, fatty acids, nucleic acids, and peptone, casamino acid, yeast extract, and soybean protein decomposition products which contain these substances may be used as the organic micronutrients. When an auxotrophic mutant strain that requires an amino acid etc. for growth is used, such a required nutrient is preferably added.

Phosphates, magnesium salts, calcium salts, iron salts, manganese salts, and so forth can be used as inorganic salts. Aerobic culturing is performed by controlling the fermentation temperature to 20 to 45°C and adjusting the pH of the culture medium to 5 to 9. When the pH decreases during the culture, the medium is neutralized by adding alkali such as calcium carbonate or ammonia gas. Culture for about 10 to about 120 hours results in accumulation of a marked amount of L-glutamic acid in the medium, which is produced via a biosynthesis pathway which utilize pyruvic acid as an intermediate.

When the coryneform bacterium used for the present invention is a coryneform bacterium which produces L-glutamic acid under a condition which induces L-glutamic acid production, such as biotin restricted condition, surfactant added condition and penicillin added condition, the medium is preferably adjusted to be under any of such L-glutamic acid production conditions. Herein, concentration of the above substances in a medium is as follows. The concentration of biotin is less than 30 µg/L, preferably less than 20 µg/L, more preferably less than 10 µg/L, and the medium may not contain biotin at all. The concentration of penicillin in the medium is not less than 0.1 U/ml, preferably not less than 0.2 U/ml, and more preferably not less than 0.4 U/ml. The concentration of surfactants in the medium is not less than 0.5 g/L, preferably not less than 1 g/L, and more preferably not less than 2 g/L. However, any concentration of these substances can be applied so long as L-glutamic acid production is induced. When antibiotic or surfactant is added to the medium, it is preferable that the medium contains sufficient amount of biotin. In such a case, it is preferable that the medium contains more than 50 µg/L, preferably more than 100 µg/L, and more preferably more than 200 µg/L of biotin.

Furthermore, the culture can also be performed with precipitating L-glutamic acid in the medium using a liquid medium adjusted to be under such a condition that L-glutamic acid is precipitated in the medium. Examples of the condition under which L-glutamic acid precipitates include pH 5.0 to 4.0, preferably pH 4.5 to 4.0, more preferably pH 4.3 to 4.0, particularly preferably pH 4.0.

L-glutamic acid may be collected from the medium after the culture by a known method. For example, the collection is attained by removing cells from the medium, and causing crystallization by concentration, ion exchange chromatography, or the like. When the culture is performed under a condition under which L-glutamic acid precipitates, L-glutamic acid precipitated in the medium can be collected by centrifugation or filtration. In this case, L-glutamic acid dissolving in the medium may be precipitated and then separated together.

### Examples

Hereafter, the present invention will be specifically explained with reference to the following Examples. However, the present invention is not limited to the Examples.

### Example 1: Construction of a vector for disrupting the sacB gene

### (A) Construction of pBS3

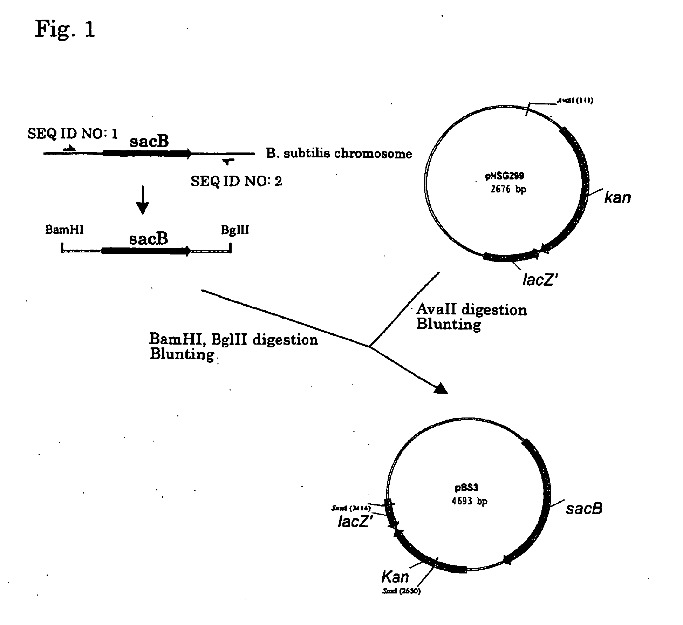

The *sacB* gene (SEQ ID NO: 7) was obtained by PCR using chromosomal DNA of *Bacillus subtilis* as a template and the oligonucleotides of SEQ ID NOS: 1 and 2 as primers. PCR was performed using LA Taq (TaKaRa) with incubation at 94°C for 5 minutes and a cycle of denaturation at 94°C for 30 second, annealing at 49°C for 30 seconds and extension at 72°C for 2 minutes, which was repeated 25 times. The PCR product was purified in a conventional manner, and then blunt-ended by digestion with Bg/II and *Bam*HI*.* This fragment was inserted into pHSG299 which had been cleaved with AvaII and blunt-ended. Using this DNA, competent cells of *Escherichia coli* JM109 (Takara Bio Inc.) were transformed, and the cells were applied to LB medium containing 25 µg/ml of kanamycin (henceforth abbreviated as Km), and cultured overnight. Then, the colonies that appeared were collected, and the individual single colonies were separated to obtain transformants. Plasmids were extracted from the obtained transformants, and a plasmid in which the objective PCR product was inserted was designated pBS3. The construction scheme of pBS3 is shown in Fig. 1.

### (B) Construction of pBS4S

Since a recognition site of the restriction enzyme SmaI is contained in the kanamycin resistance gene present in pBS3, a plasmid carrying a kanamycin resistance gene of which *SmaI* site was disrupted by nucleotide substitution not causing amino acid substitution was obtained by crossover PCR. First, PCR is performed using pBS3 as a template and the synthetic DNAs of SEQ ID NOS: 3 and 4 as primers to obtain an amplification product of the N-terminus side sequence of the kanamycin resistance gene. Further, in order to obtain an amplification product of the C-terminus side sequence of the Km resistance gene, PCR was performed using pBS3 as a template and the synthetic DNAs of SEQ ID NOS: 5 and 6 as primers. The objective PCR product could be obtained by performing PCR using Pyrobest DNA Polymerase (Takara Bio Inc.) with incubation at 98°C for 5 minutes and a cycle of denaturation at 98°C for 10 seconds, annealing at 57°C for 30 seconds and extension at 72°C for 1 minute, which was repeated 25 times. The sequences of SEQ ID NOS: 4 and 5 are partially complementary to each other, and the *SmaI* site which had been existed in these sequences was disrupted by nucleotide substitution not causing amino acid substitution. Then, in order to obtain a mutant kanamycin resistance gene fragment in which the *SmaI* site was disrupted, the aforementioned gene products of the N-terminus and C-terminus side sequences of the kanamycin resistance gene were mixed in substantially equivalent moles, and PCR was performed using this mixture as a template and the synthetic DNAs of SEQ ID NOS: 3 and 6 as primers to obtain an amplification product of the Km resistance gene introduced with a mutation. The PCR product could be obtained by performing PCR using Pyrobest DNA Polymerase (Takara Bio Inc.) with incubation at 98°C for 5 minutes and a cycle of denaturation at 98°C for 10 seconds, annealing at 57°C for 30 seconds and extension at 72°C for 1.5 minutes, which was repeated 25 times.

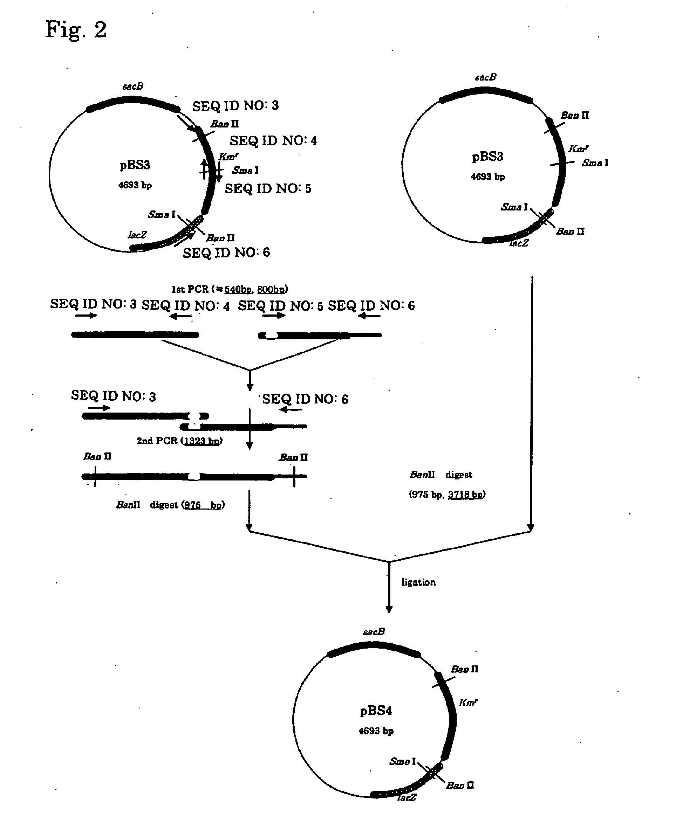

The PCR product was purified in a conventional manner, then digested with *BanII,* and inserted into the *BanII* site of pBS3 described above. Competent cells of *Escherichia coli* JM109 (Takara Bio Inc.) were transformed with the obtained DNA, and the cells were applied on LB medium containing 25 µg/ml of kanamycin, and cultured overnight. Then, single colony were separated from the emerged colonies to obtain transformants. Plasmids were extracted from the obtained transformants, and a plasmid in which the objective PCR product was inserted was designated pBS4S. The construction scheme of pBS4S is shown in Fig. 2.

### Example 2: Preparation of gluX-deficient strain of C. glutamicum ATCC 13869

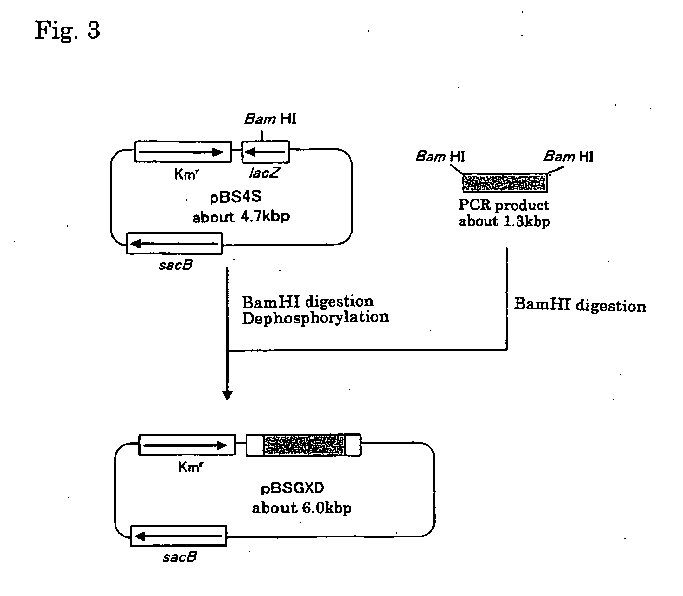

A plasmid for deleting the *gluX* gene was prepared. A chromosome was extracted from C. *glutamicum* ATCC 13869 using Bacterial Genomic DNA Purif. Kit (MS Techno Systems), and PCR was performed using this chromosome as a template and a combination of the primers of SEQ ID NOS: 9 and 10, and a combination of the primers of SEQ ID NOS: 11 and 12 to amplify fragments of about 650 bp, respectively. PCR was performed using Ex Taq (Takara Bio Inc.) with a cycle of denaturation at 94°C for 30 seconds, annealing at 55°C for 10 seconds and extension at 72°C for 1 minute, which was repeated 25 times. The primers of SEQ ID NOS: 9 and 10 were designed to amplify the region of the nucleotide numbers 401 to 1040 of SEQ ID NO: 16, and the primers of SEQ ID NOS: 11 and 12 were designed to amplify the region of the nucleotide numbers 1241 to 1920 of SEQ ID NO: 16. Then, PCR was performed using a solution prepared by mixing these two fragments as templates and the primers of SEQ ID NOS: 13 and 14 to amplify a fragment of about 1.3 kb. PCR was performed using Ex Taq (Takara Bio Inc.) with a cycle of denaturation at 94°C for 30 seconds, annealing at 55°C for 10 seconds and extension at 72°C for 1.5 minutes, which was repeated 25 times. The primers of SEQ ID NOS: 13 and 14 were designed to add a *Bam*HI sequence to at the 5' end, and amplify the region of the nucleotide numbers 441 to 1870 of SEQ ID NO: 16 (including deletion of the region of the nucleotide numbers 1041 to 1240). The amplified fragment was completely digested witch *Bam*HI, and ligated to the pBS4S vector described in Example 1 which had been similarly completely digested with BamHI (Ligation Kit Ver. 2 (Takara Bio Inc.) was used) to construct a vector for *gluX* disruption, pBSGXD. The construction scheme is shown in Fig. 3.

pBSGXD was introduced into *C*. *glutamicum* ATCC 13869 by the electric pulse method (Japanese Patent Laid-open No. 2-207791), and the cells were applied to the CM-Dex agar medium (5 g/l of glucose, 10 g/l of polypeptone, 10 g/l of yeast extract, 1 g/l of KH₂PO₄, 0.4 g/l of MgSO₄· 7H₂O, 0.01 g/l of FeSo₄· 7H₂O,0.01 g/l of MnSO₄· 4-5H₂O,3 g/l of urea, 1.2 g/l of soy bean protein hydrolysate, 20 g/l of agar, adjusted to pH 7.5 with NaOH) containing 25 µg/ml of kanamycin. Culture was performed at 31.5°C, and then it was confirmed by PCR that the grown strain was a one time-recombinant strain in which pBSGXD was incorporated into the chromosome by homologous recombination. Whether a candidate strain is a one time-recombinant strain can be easily confirmed by performing PCR using a chromosome of the strain as a template, a specific sequence on pBS4S (SEQ ID NO: 15) and a sequence on the chromosome (SEQ ID NO: 9) as primers. Since the sequence of pBS4S is not present on chromosome of a non-recombinant strain, any fragment amplified by PCR does not appear for the non-recombinant strain, and therefore discrimination becomes possible.

The obtained once-recombinant strain was cultured at 31.5°C for one whole day in the CM-Dex liquid medium containing 25 µg/ml of kanamycin, and this medium was appropriately diluted, and applied to S 10 plates (having a composition of the CM-Dex medium mentioned above in which 5 g/l of glucose was replaced with 100 g/l of sucrose). Several strains grown on the S 10 plates and showing kanamycin sensitivity were selected, and PCR was performed using the chromosomes of these strains as a template and the sequences of SEQ ID NOS: 13 and 14 as primers to confirm that one strain among them was an objective *gluX* deficient strain. Since the region of the nucleotide numbers 1041 to 1240 is deleted in the deficient strain, the amplified fragment is shorter than that of the non-deficient strain, and therefore discrimination becomes possible. The deficient strain obtained as described was designated ATCC 13869 ΔgluX.

### Example 3: Measurement of glutamic acid production amount of ATCC138690ΔgluX strain

Glutamic acid production ability of the ATCC13869ΔgluX strain was examined by performing culture using a Sakaguchi flask. The ATCC 13869 and ATCC 13869ΔgluX strains were cultured at 31.5°C on the CM-2B agar medium (10 g/l of polypeptone, 5 g/l of yeast extract, 5 g/l of NaCl, 10 µg/l of biotin, 20 g/l of agar, adjusted to pH 7.0 with KOH) for one whole day, and inoculated into 20 ml of a seed culture medium (50 g/l of glucose, 30 g/l of ammonium sulfate, 1 g/l of KH₂PO₄, 0.4 g/l of MgSO₄ · 7H₂O, 0.01 g/l of FeSO₄ · 7H₂O, 0.01 g/l of MnSO₄ · 4-5H₂O, 200 µg/l of vitamin B1, 0.48 g/l of soy bean protein hydrolysate, 300 µg/l of biotin, adjusted to pH 8.0 with KOH). One g of calcium carbonate which had been subjected to dry heat sterilization beforehand was added to the medium, and then culture was performed at 31.5°C with shaking at a velocity of 115 rpm. After it was confirmed that the total sugar was completely consumed, 1 ml of the seed culture medium was inoculated into 20 ml of a main culture medium (50 g/l of glucose, 30 g/l of ammonium sulfate, 1 g/l of KH₂PO₄, 0.4 g/l of MgSO₄ · 7H₂O, 0.01 g/l of FeSO₄ · 7H₂O, 0.01 g/l of MnSO₄ · 4-5H₂O,200 µg/l of vitamin B1, 0.48 g/l of soy bean protein hydrolysate, 300 µg/l of biotin, adjusted to pH 8.0 with KOH), followed by adding 1 g of calcium carbonate which had been subjected to dry heat sterilization beforehand, and then culture was performed at 31.5°C with shaking at a velocity of 115 rpm. After 2.5 hours from the start of the culture, 4 g/l of Tween 40 (Sigma) was added. The cell amounts (absorption was measured at 620 nm), the accumulated glutamic acid amounts, and the remained saccharide amounts after 17.5 hours are shown in Table 3.

It was confirmed that the glutamic acid accumulation by the ATCC13 869ΔgluX strain increased compared with that of ATCC13869, and thus it was confirmed that deletion of the *gluX* gene was effective for improvement in glutamic acid production.

**Table 1**

| | OD620nm | Glutamic acid (g/l) | Remaining saccharide (g/l) |
|---|---|---|---|
| ATCC13869 | 44.88 ± 0.77 | 21.5 ± 0.4 | 0.0 |
| ATCC13869ΔgluX | 43.81 ± 0.46 | 23.3 ± 0.1 | 0.0 |

### Industrial Applicability

According to the present invention, fermentation yield of L-glutamic acid can be increased in a method for producing L-glutamic acid by fermentation using a coryneform bacterium. Moreover, the present invention can be used for breeding of L-glutamic acid producing bacteria.

## Claims

1. A method for producing L-glutamic acid, which comprises culturing a coryneform bacterium having L-glutamic acid producing ability in a medium to produce and accumulate L-glutamic acid, and collecting L-glutamic acid from the medium, wherein said coryneform bacterium is modified so that the *gluX* gene on the chromosome is disrupted and
wherein said *gluX* gene is selected from the group consisting of:
(a) a DNA which comprises the nucleotide sequence consisting of 1001 to 1279 of SEQ ID NO: 16, and
(b) a DNA which is able to hybridize with the nucleotide sequence consisting of 1001 to 1279 of SEQ ID NO: 16 under stringent conditions comprising washing at 0.1 x SSC, 0.1 % SDS at 60° C, and encodes a protein having a function of improving production amount of L-glutamic acid when the DNA is inactivated on a chromosome.

2. The method according to claim 1, wherein said *gluX* gene encodes a protein selected from the group consisting of:
(A) a protein comprising the amino acid sequence of SEQ ID NO: 17, and
(B) a protein comprising the amino acid sequence of SEQ ID NO: 17 including substitution, deletion, insertion, or addition of one to 10 amino acid residues and having a function of improving production amount of L-glutamic acid when the DNA is inactivated on a chromosome.

3. The method according to according to claims 1 or 2, wherein said coryneform bacterium is *Corynebacterium glutamicum.*

## Patentansprüche

1. Verfahren zum Herstellen von L-Glutaminsäure, das umfasst: das Kultivieren eines coryneformen Bakteriums mit der Fähigkeit, L-Glutaminsäure herzustellen, in einem Medium, um L-Glutaminsäure herzustellen und anzusammeln, sowie Eintreiben der L-Glutaminsäure aus dem Medium wobei das coryneforme Bakterium so modifiziert ist, dass das *gluX*-Gen des Chromosoms unterbrochen ist und
wobei das *gluX*-Gen ausgewählt wird aus der Gruppe bestehend aus:
(a) einer DNA, die die Nucleotidsequenz bestehend aus 1001 bis 1279 von SEQ ID Nr.: 16 umfasst, und
(b) einer DNA, die befähigt ist mit der Nucleotidsequenz bestehend aus 1001 bis 1279 der SEQ ID Nr.: 16 unter stringenten Bedingungen zu hybridisieren, welche das Waschen mit 0,1 x SSC, 0,1 % SDS bei 60°C umfassen, und die für ein Protein kodiert, das eine Funktion bei der Verbesserung der Herstellungsmenge von L-Glutaminsäure hat, wenn die DNA auf einem Chromosom inaktiviert wird.

2. Verfahren gemäß Anspruch 1, wobei das *gluX*-Gen für ein Protein ausgewählt aus der Gruppe bestehend aus:
(A) einem Protein, umfassend die Aminosäuresequenz der SEQ ID Nr.: 17, und
(B) einem Protein, umfassend die Aminosäuresequenz der SEQ ID Nr.: 17 einschließlich Substitution, Deletion, Insertion oder Addition von ein bis 10 Aminosäureresten und mit einer Funktion zur Verbesserung der Produktionsmenge von L-Glutaminsäure, wenn die DNA auf einem Chromosom inaktiviert ist.
kodiert.

3. Verfahren Gemäß Anspruch 1 oder 2, wobei das coryneforme Bakterium *Corynebacterium glutamicum* ist.

## Revendications

1. Procédé pour produire de l'acide L-glutamique qui comprend la culture d'une bactérie corynéforme ayant une aptitude à produire de l'acide L-glutamique dans un milieu pour produire et accumuler de l'acide L-glutamique, et la collecte de l'acide L-glutamique à partir du milieu, où ladite bactérie corynéforme est modifiée de sorte que le gène *gluX* sur le chromosome est interrompu et où ledit gène *gluX* est choisi dans le groupe consistant en :
(a) un ADN qui comprend la séquence nucléotidique consistant en 1001 à 1279 de SEQ ID NO : 16, et
(b) un ADN qui est capable de s'hybrider avec la séquence nucléotidique consistant en 1001 à 1279 de SEQ ID NO : 16 dans des conditions stringentes comprenant un lavage à 0,1 x SSC, 0,1 % de SDS à 60°C, et code une protéine ayant une fonction d'amélioration de la quantité de production d'acide L-glutamique quand l'ADN est inactivé sur un chromosome.

2. Procédé selon la revendication 1 où ledit gène *gluX* code une protéine choisie dans le groupe consistant en :
(A) une protéine comprenant la séquence d'aminoacides de SEQ ID NO : 17, et
(B) une protéine comprenant la séquence d'aminoacides de SEQ ID NO : 17 incluant une substitution, délétion, insertion ou addition de un à 10 résidus d'aminoacides et ayant une fonction d'amélioration de la quantité de production d'acide L-glutamique quand l'ADN est inactivé sur un chromosome.

3. Procédé selon la revendication 1 ou 2 où ladite bactérie corynéforme est *Corynebacterium glutamicum.*
